# DEMANDE DE BREVET EUROPEEN

(11) **EP 4 821 447 A1**
(43) Date de publication de la demande: **14.10.2026**
(21) Numéro de dépôt: 25169215.8
(22) Date de dépôt: 08.04.2025
(51) Int. Cl.: A61B 17/04

(54) **INSTRUMENT DE LIGATURE D'UN ORGANE AU MOYEN D'UN FIL DE LIGATURE ET PROCÉDÉ DE MISE EN OEUVRE DE CET INSTRUMENT**

(71) Demandeur: Assistance Publique - Hôpitaux de Paris, 75012 Paris (FR)
(72) Inventeur: CAMPOS, Guillaume, 67000 Strasbourg (FR); GLOMAUD, Adrien, 75013 Paris (FR)
(74) Mandataire: Cabinet Camus Lebkiri

(57) **Abrégé**

Un aspect de l'invention concerne un instrument de ligature (100) d'un organe (10) d'un corps humain ou animal au moyen d'un fil de ligature (20), comportant :
- un bâti (130) sur lequel est montée au moins une tête de bouclage (150),
- un actionneur (140) assurant une rotation de la tête de bouclage, ladite rotation mettant le fil de ligature en forme de boucle autour de ladite tête de bouclage,
- une crosse (200) fixée à l'extérieur du bâti et maintenant une extrémité libre du fil de ligature à proximité de l'organe à ligaturer, et
- un dispositif de nouage (300), tubulaire, se déplaçant au travers de la tête de bouclage et assurant la préhension de l'extrémité libre du fil de ligature et la formation d'un nœud.

Un autre aspect de l'invention concerne un procédé de mise en œuvre de l'instrument de ligature.

## Description

### DOMAINE TECHNIQUE DE L'INVENTION

La présente invention concerne un instrument pour ligaturer automatiquement un organe ou une structure du corps humain ou animal au moyen d'un fil de ligature. L'invention concerne aussi le procédé de ligature mis en œuvre par cet instrument.

L'invention trouve des applications dans le domaine chirurgical et notamment dans tous les domaines de la chirurgie pouvant nécessiter la ligature d'un ou plusieurs organes ou structures du corps humain ou animal.

### ARRIERE-PLAN TECHNOLOGIQUE DE L'INVENTION

Dans la plupart des domaines de la chirurgie, humaine ou animale, comme les chirurgies cardiaque, vasculaire, viscérale, digestive, gynécologique, etc., le chirurgien est souvent amené à ligaturer une structure ou un organe du corps, comme les artères, les veines, les lymphatiques, l'appendice, les canaux biliaires, etc. Une ligature consiste à comprimer, de façon hermétique, une structure ou un organe en l'entourant, en le serrant et en l'attachant avec un fil chirurgical. Pour cela, le chirurgien doit effectuer manuellement, autour de l'organe ou de la structure ciblée, au moins un nœud et, généralement plusieurs nœuds successifs, avec un fil chirurgical, appelé aussi fil de ligature ou fil de suture.

Cependant, cette méthode est longue et fastidieuse. Elle nécessite en général plusieurs nœuds de ligature (appelés plus simplement nœuds) dont le serrage peut être irrégulier car totalement dépendant de la main du chirurgien.

Pour répondre à ces problèmes, des techniques ont été développées dans lesquelles les nœuds en fil chirurgical sont remplacés par des clips chirurgicaux. Ces clips chirurgicaux sont posés au moyen d'une pince à clips, ce qui permet une pose plus rapide et plus régulière. Cependant, ces clips chirurgicaux présentent l'inconvénient d'être métalliques et par conséquent non résorbables. Ils présentent aussi l'inconvénient d'être peu fiables car, non seulement ils peuvent sectionner les structures ou organes au lieu de les ligaturer, mais ils ont également tendance à se détacher facilement des structures ou organes ciblés.

Il existe, par ailleurs, une alternative à la ligature des structures ou organes, à savoir l'électrocoagulation. Cette technique d'électrocoagulation consiste à couper les tissus et coaguler les vaisseaux sanguins de ces tissus au moyen d'un dispositif d'électrocoagulation. Un dispositif d'électrocoagulation comporte généralement une source électrique à laquelle sont reliées des électrodes, l'une de ces électrodes transmettant une chaleur suffisamment élevée pour assurer la coagulation des vaisseaux sanguins. Cependant, cette technique d'électrocoagulation a l'inconvénient d'être dépendante d'une source électrique et de présenter un coût élevé.

Il existe donc un réel besoin d'une technologie permettant une ligature simple, rapide et régulière des structures ou organes ciblés.

### RESUME DE L'INVENTION

Pour répondre aux problèmes évoqués ci-dessus du caractère long, fastidieux et irrégulier d'une ligature classique par fil chirurgical, le demandeur propose un instrument miniature, adapté aux problématiques de miniaturisation spécifiques au domaine chirurgical et permettant une réalisation simplifiée, rapide et régulière d'un ou plusieurs nœuds de ligature.

Selon un premier aspect, l'invention concerne un instrument de ligature d'un organe d'un corps humain ou animal au moyen d'un fil de ligature, comportant :
- un bâti sur lequel est montée au moins une tête de bouclage supportant le fil de ligature,
- un actionneur assurant une rotation de la tête de bouclage par rapport au bâti, la rotation de la tête de bouclage mettant le fil de ligature en forme de boucle autour de ladite tête de bouclage,
- une crosse fixée à l'extérieur du bâti et maintenant une extrémité libre du fil de ligature à proximité de l'organe à ligaturer, et
- un dispositif de nouage, tubulaire, se déplaçant au travers de la tête de bouclage et assurant la préhension de l'extrémité libre du fil de ligature et la formation d'un nœud par passage de ladite extrémité libre du fil de ligature au travers de la boucle préalablement formée.

Cet instrument de ligature, appelé plus simplement instrument, présente l'avantage de former facilement et rapidement, autour de l'organe ciblé, des nœuds de ligature uniformes dont le serrage est régulier. Il présente également, par rapport aux clips, les avantages d'une ligature par fil chirurgical, c'est-à-dire par un fil résorbable, avec peu de risque de rupture de l'organe. Il présente aussi l'avantage d'être totalement mécanique et, par conséquent, indépendant de toute source d'énergie électrique.

Dans la suite de la description, on appellera « organe », toute structure, organe, ou tissu du corps humain ou animal susceptible d'être ligaturé.

On appellera indifféremment « fil de ligature », « fil de suture », « fil chirurgical » ou simplement « fil », tout fil classiquement utilisé dans les domaines chirurgicaux pour une ligature à base de fil (par opposition aux clips chirurgicaux).

On appellera « extrémité proximale », la zone ou l'environnement de l'instrument le plus proche de la main du chirurgien et donc du dispositif de commande de cet instrument, par opposition à « l'extrémité distale » qui est la zone ou l'environnement de l'instrument le plus proche de l'organe à ligaturer et donc le plus éloigné de la main du chirurgien pendant l'intervention de ligature. Par analogie, un déplacement « vers/dans la direction proximale » signifie vers la main du chirurgien et un déplacement « vers/dans la direction distale » signifie vers l'organe ciblé. Par définition, les directions proximale et distale sont des directions longitudinales qui s'étendant parallèlement à l'axe central XX de l'instrument de ligature. Par opposition, la direction transversale est une direction perpendiculaire à la direction longitudinale.

Avantageusement, l'instrument de ligature selon le premier aspect comporte au moins deux têtes de bouclage alignées, assurant la formation d'au moins deux boucles de fil de ligature, alignées, formées par un unique actionnement de l'actionneur, le dispositif de nouage assurant la formation d'au moins deux nœuds par passage de l'extrémité libre du fil de ligature au travers de chacune des deux boucles.

Ce mode de réalisation présente l'avantage de permettre la réalisation automatique de plusieurs nœuds de ligature identiques, le nombre classique de nœuds pour une ligature étant de trois.

On entend par « bouclage » le fait de former une boucle avec le fil de ligature, c'est-à-dire de donner au fil une forme sensiblement ronde ou oblongue, le fil s'étendant dans une direction transversale, sur deux hauteurs distinctes, entre deux parois parallèles d'un dispositif appelé tête de bouclage, et s'entrelaçant de sorte qu'une portion du fil hors boucle s'étend dans la direction distale et l'autre portion du fil hors boucle s'étend dans la direction proximale.

Outre les caractéristiques qui viennent d'être évoquées dans les paragraphes précédents, l'instrument de ligature selon le premier aspect de l'invention peut présenter une ou plusieurs caractéristiques complémentaires parmi les suivantes, considérées individuellement ou selon toutes les combinaisons techniquement possibles :
- la tête de bouclage, montée pivotante dans le bâti, comporte une double agrafe et un double croc dans le prolongement l'un de l'autre, la rotation de la double agrafe et du double croc entraînant une rotation et un guidage du fil de ligature lui assurant une mise en forme de boucle.
- la tête de bouclage comporte deux lames, parallèles et symétriques l'une et l'autre par rapport à un plan central longitudinal, chaque lame comportant une section sensiblement trapézoïdale dont une première face latérale étendue forme une agrafe et une deuxième face latérale étendue forme un croc, l'agrafe et le croc étant coplanaires.
- chaque lame comporte une face supérieure sensiblement droite et une face inférieure à partir de laquelle s'étend longitudinalement une fente de retenue du fil de ligature.
- les deux lames de la double agrafe et du double croc forment une gorge s'étendant suivant un axe longitudinal, dans laquelle se déplace le dispositif de nouage.
- l'actionneur comporte un dispositif de pivotement de la tête de bouclage entraînant la rotation d'un ¾ de tour de la tête de bouclage depuis une position initiale perpendiculaire à un axe longitudinal vers une position finale parallèle à l'axe longitudinal
- l'actionneur comporte une platine, mobile en translation, munie d'une rainure longitudinale recevant la tête de bouclage et en ce que le dispositif de pivotement comporte une crémaillère.
- la rainure longitudinale de la platine comporte un étrécissement et la crémaillère comporte, au voisinage de l'étrécissement, au moins une dent escamotable assurant une indexation angulaire de la tête de bouclage.
- le dispositif de nouage comporte un crochet monté coulissant à l'intérieur d'un premier fourreau et assurant la préhension de l'extrémité libre du fil de ligature et la formation du nœud par passage de l'extrémité dudit fil à travers la boucle formée par la tête de bouclage.
- le crochet comporte une attache recourbée, montée à une extrémité distale d'une tringle sensiblement rectiligne.
- le crochet comporte une tringle coudée, équipée d'un renflement à son extrémité distale.
- le dispositif de nouage comporte une broche poussoir dont une extrémité distale est équipée de deux ailettes, escamotables à l'intérieur d'un deuxième fourreau, les deux ailettes formant une fourche de décrochage du nœud.
- le crochet, le premier fourreau, la broche poussoir et le deuxième fourreau sont montés concentriques les uns dans les autres de sorte à former un unique outil déplaçable au travers de la tête de bouclage.
- la broche poussoir comporte une tige creuse formant le premier fourreau dans lequel coulisse le crochet.
- le bâti comporte, à une extrémité distale, un clapet d'alignement du fil de ligature autorisant le déplacement du fil de ligature dans la direction distale et le blocage dudit fil de ligature dans la direction proximale.

Selon un deuxième aspect, l'invention concerne un procédé de mise en œuvre de l'instrument de ligature selon le premier aspect. Ce procédé comporte les opérations suivantes :
a) mise en place d'un fil de ligature à l'intérieur de l'instrument de ligature, le fil traversant la tête de bouclage avec une tension prédéterminée, une extrémité libre du fil étant installée sur une crosse à une extrémité distale dudit instrument de ligature et une bobine de fil étant positionnée au voisinage d'une extrémité proximale dudit instrument de ligature,
b) commande de l'actionneur pour entraîner la tête de bouclage en rotation de sorte à former une boucle du fil de ligature,
c) déplacement du dispositif de nouage au travers de la boucle formée par la tête de bouclage en direction de l'extrémité distale de l'instrument de ligature et préhension de l'extrémité libre du fil,
d) déplacement, en direction de l'extrémité proximale de l'instrument de ligature, du dispositif de nouage et de l'extrémité libre du fil de ligature au travers de la boucle de fil de ligature pour former un nœud,
e) déplacement du dispositif de nouage au travers de la tête de bouclage, en direction de l'extrémité distale de l'instrument de ligature, et décrochage du nœud hors de la tête de bouclage,
f) déplacement complémentaire du dispositif de nouage en direction de l'organe à ligaturer et serrage du nœud contre ledit organe à ligaturer.

Ce procédé présente l'avantage d'être simple à mettre en œuvre puisqu'il nécessite uniquement, de la part du chirurgien, les commandes de déplacement de l'actionneur et du dispositif de nouage de l'instrument.

Avantageusement, le procédé selon le deuxième aspect, dans lequel l'instrument de ligature comporte au moins deux têtes de bouclage, est caractérisé par le fait que les opérations e) et f) sont réitérées pour chaque nœud formé par l'une des têtes de bouclage de sorte que chaque nœud est serré indépendamment l'un de l'autre.

### BREVE DESCRIPTION DES FIGURES

D'autres avantages et caractéristiques de l'invention apparaîtront à la lecture de la description qui suit, illustrée par les figures dans lesquelles :
La figure 1 représente schématiquement un exemple d'instrument de ligature selon l'invention, selon une première vue en perspective depuis l'extrémité proximale de l'instrument (dessin A) et selon une deuxième une vue en perspective depuis l'extrémité distale de l'instrument (dessin B).
La figure 2 représente schématiquement un exemple de la partie intérieure de l'instrument de ligature de l'invention, selon une vue de dessus (dessins A) et une vue en perspective (dessins B) de cette partie intérieure de l'instrument.
La figure 3 représente schématiquement un exemple de têtes de bouclage (dessins A et B) montées sur la platine de support de l'instrument de ligature dans une première et une deuxième position (dessins C et D).
La figure 4 représente schématiquement une partie de la tête de bouclage dans plusieurs positions, depuis la position initiale de mise en place du fil de ligature jusqu'à la position finale d'obtention d'une boucle.
La figure 5 représente schématiquement plusieurs exemples du crochet du dispositif de nouage de l'instrument de ligature (dessins A et B), dans plusieurs positions (dessin C).
La figure 6 représentent schématiquement plusieurs exemples de la broche-poussoir du dispositif de nouage de l'instrument de ligature (dessins A et C), monté sur la platine de support (dessin B) ou en interaction avec un nœud (dessin D).
La figure 7 représente schématiquement plusieurs exemples d'un dispositif de nouage imbriquant un crochet et une broche-poussoir (dessins A, B et C).
La figure 8 représente de façon fonctionnelle un exemple du procédé de mise en œuvre de l'instrument de ligature selon les figures 1 et 2.
La figure 9A, la figure 9B, la figure 9C, la figure 9D et la figure 9E représentent schématiquement différents positionnements du dispositif de nouage lors de la formation et du serrage d'un nœud de ligature.
La figure 10A, la figure 10B, la figure 10C, la figure 10D et la figure 10E représentent schématiquement différents positionnements du dispositif de nouage au sein de l'instrument de ligature lors de la réalisation et du serrage d'un nœud.

Sur les figures, les éléments identiques sont repérés par des références identiques. Pour des questions de lisibilité des figures, les échelles de taille entre éléments représentés ne sont pas respectées.

### DESCRIPTION DETAILLEE

Des exemples de réalisation d'un instrument de ligature d'un organe et de son procédé de mise en œuvre sont décrits en détail ci-après, en référence aux dessins annexés. Ces exemples illustrent les caractéristiques et avantages de l'invention. Il est toutefois rappelé que l'invention ne se limite pas à ces exemples.

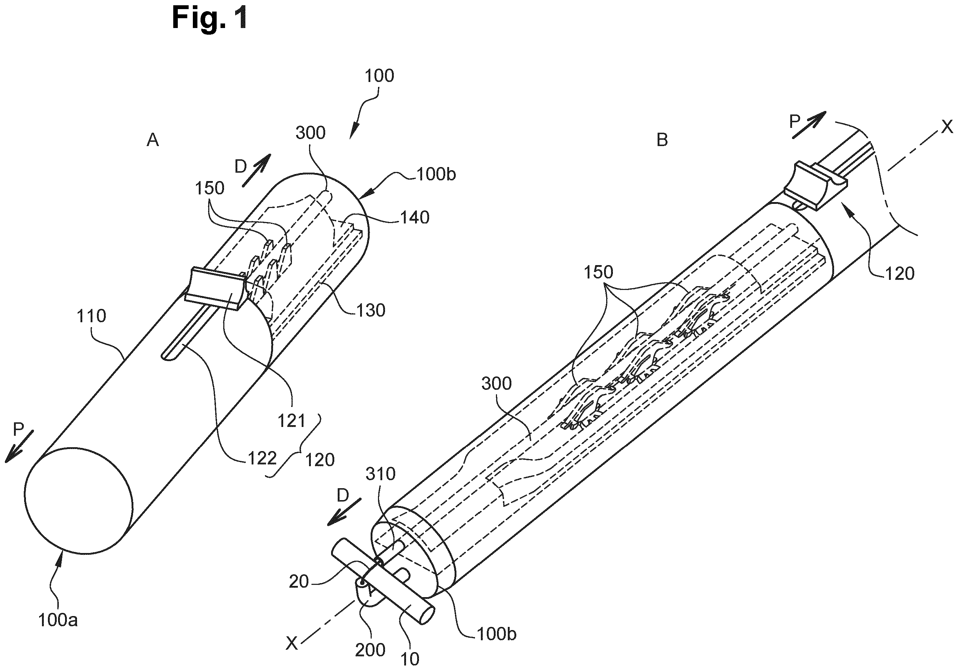

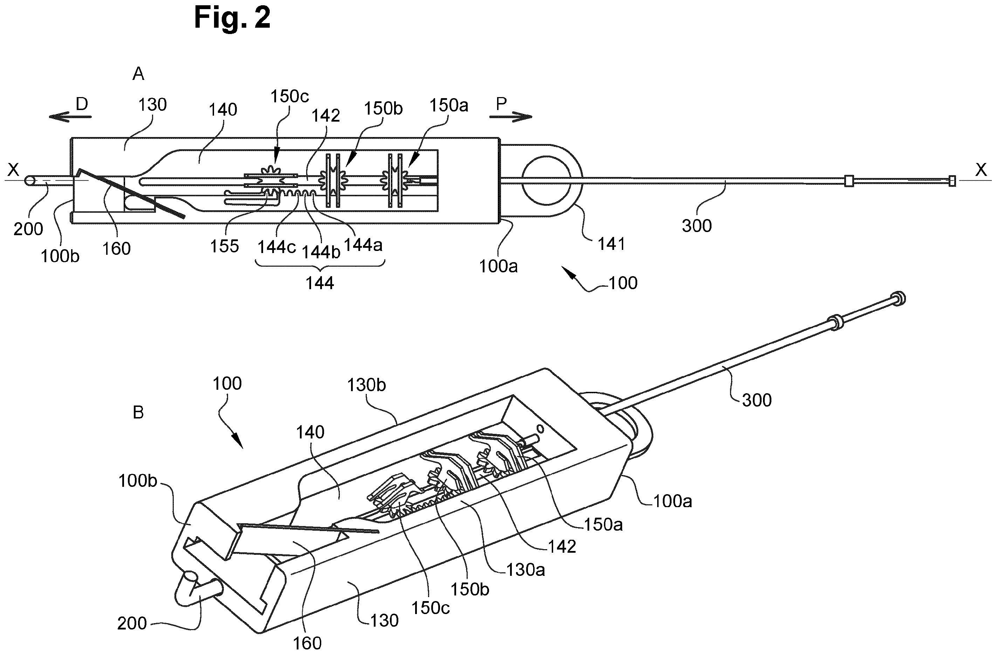

Un exemple d'un instrument de ligature d'un organe du corps humain ou animal est représenté selon des vues en perspective sur la figure 1. Cet instrument de ligature 100 s'étend depuis une extrémité proximale 100a jusqu'à une extrémité distale 100b. La vue A de la figure 1 montre l'instrument 100 dans une perspective de l'extrémité proximale 100a vers l'extrémité distale 100b. La vue B de la figure 1 montre le même instrument 100 dans une perspective de l'extrémité distale 100b vers l'extrémité proximale 100a. L'instrument 100 comporte un étui 110 s'étendant selon un axe central XX et dans lequel sont logés la majorité des pièces et dispositifs permettant la ligature de l'organe 10, appelés pièces et dispositifs intérieurs. Cet étui 110 forme une enveloppe de protection pour lesdites pièces et dispositifs intérieurs ainsi que pour le fil de ligature 20 lors de la formation des nœuds. L'étui 110, représenté partiellement transparent sur la figure 1 pour une meilleure compréhension de l'invention, peut être réalisé dans un ou plusieurs matériaux plastiques et/ou métallique et/ou dans tout autre matériau, transparent ou opaque, ayant des propriétés compatibles avec le domaine de la chirurgie. L'étui 110 est avantageux dans la mesure où il protège les pièces et dispositifs intérieurs de l'instrument, mais non obligatoire. Un exemple de l'instrument de ligature 100 sans étui, représenté sur la figure 2, sera décrit plus loin.

Selon certains modes de réalisation de l'invention, l'instrument de ligature 100 est équipé d'un dispositif de commande 120 utilisé par le chirurgien pour commander le déplacement des différentes pièces et dispositifs intérieurs afin de former des nœuds de ligature de l'organe 10. Dans l'exemple de la figure 1, le dispositif de commande 120 comporte une glissière 122, logée dans l'étui 110 et dans laquelle coulisse un curseur 121. La glissière est formée, par exemple, d'une cavité longitudinale, préférentiellement de section rectangulaire ou ovale, dans laquelle se déplace le curseur 121. Le curseur 121 est relié à certains des dispositifs et pièces intérieurs, par exemple un actionneur et/ou un dispositif de nouage décrits ultérieurement, pour permettre leur déplacement à l'intérieur de l'étui 110. Dans l'exemple de la figure 1, le dispositif de commande 120 est mobile en translation et se déplace parallèlement à l'axe central XX, appelé aussi axe longitudinal.

Bien entendu, l'homme du métier comprendra que des dispositifs de commande, autres que celui représenté sur la figure 1, par exemple un ou plusieurs boutons-poussoirs, un système rotatif à came, un système de levier associé à une crémaillère et un cliquet ou encore un système de pignonnerie associé à un curseur, peuvent être utilisés à la place du dispositif de commande 120 de la figure 1, le dispositif de commande ayant pour fonction principale de commander l'actionneur et/ou le dispositif de nouage 300 décrit par la suite.

L'instrument de ligature 100 comporte une crosse 200 fixée à l'extrémité distale dudit instrument de ligature. La crosse 200 peut être fixée à l'étui 110, ou bien à un bâti ou un boîtier décrits par la suite. Dans l'exemple de la figure 2, la crosse 200 est positionnée à l'extrémité distale 100b du boîtier 130. Cette crosse 200 s'étend à l'extérieur de l'étui 110 (ou du bâti ou du boîtier 130) dans la direction distale D. Elle a pour fonction, d'une part, de maintenir au moins partiellement l'organe à ligaturer 10 en désignant la zone dudit organe où doit être réalisée la ligature et, d'autre part, de maintenir l'extrémité libre du fil de ligature 20 pendant une partie de l'intervention de ligature. En effet, en début d'intervention, la crosse 200 est positionnée sous l'organe à ligaturer 10, appelé aussi organe ciblé, et le fil de ligature 20 est installé de sorte que son extrémité soit maintenue dans la crosse 200. Pour cela, la crosse 200 peut comporter, à son extrémité libre située à l'extérieur de l'étui 110, une rainure centrale, un crochet ou tout autre moyen d'attache assurant le maintien de l'extrémité du fil 20.

Les dispositifs et pièces intérieurs à l'étui 110 sont représentés au moins en partie sur la figure 2. Comme évoqué précédemment, l'instrument de ligature 100 peut ne pas comporter d'étui 110 ou comporter uniquement un étui partiel monté autour d'un bâti ou d'un boîtier 130. Dans ce cas, l'instrument de ligature 100 comporte les dispositifs et pièces représentés sur cette figure 2. Dans le mode de réalisation où l'instrument de ligature 100 comporte un étui, l'étui peut être monté autour de l'instrument 100 ou uniquement du bâti ou du boîtier 130 représenté sur la figure 2.

Qu'il comprenne ou non un étui, l'instrument de ligature 100 comporte un bâti ou un boîtier 130 tel que représenté sur la figure 2, ayant une extrémité proximale 100a et une extrémité distale 100b. L'instrument de ligature pouvant être réalisé avec ou sans étui de protection 110, les expressions « extrémité proximale 100a » et « extrémité distale 100b » seront comprises comme étant la partie de l'instrument 100, par exemple le bâti ou le boîtier 130, respectivement, la plus proche et la plus éloignée de la main du chirurgien.

Le bâti a notamment pour fonction de supporter les têtes de bouclage 150 (décrites en détail par la suite) ainsi que la crosse 200 et d'assurer la connexion, ou liaison mécanique, entre les têtes de bouclage et un actionneur permettant le pivotement de ces têtes de bouclage. Dans l'exemple de la figure 2, et dans la suite de la description, le bâti se présente sous la forme d'un boîtier 130, étant entendu que le bâti peut prendre d'autres formes que celles d'un boîtier, par exemple un socle, une embase ou toute autre structure ayant une ou plusieurs surfaces permettant la fixation ou le maintien des pièces et dispositifs de l'instrument de ligature.

Dans le boîtier 130, sont montées une ou plusieurs têtes de bouclage 150 en liaison mécanique avec l'actionneur. Dans les exemples des figures 2 et suivantes, l'actionneur sera décrit dans le cas où il comporte une platine de support 140, mobile en translation, et un système à crémaillère 144, étant entendu que l'actionneur peut également être un système à chaîne ou une pignonnerie, la fonction de l'actionneur étant d'entraîner les têtes de bouclage en rotation par rapport au bâti. Plus précisément, dans l'exemple de la figure 2, le boîtier 130 comporte une platine de support 140 montée mobile en translation et s'étendant, dans son état initial, de l'extrémité proximale 100a à l'extrémité distale 100b du boîtier 130. La platine de support 140 est mobile au sein du boîtier 130 et peut être déplacée vers la direction proximale P et/ou vers la direction distale D. Pour cela, la platine de support 140 est montée sur le boîtier 130 au moyen d'un système de glissières latérales formé dans les parois latérales 130a, 130b du boîtier (non visibles sur les figures) ou de tout autre moyen permettant un déplacement en translation bidirectionnel de la platine de support 140 par rapport au boîtier 130. La platine de support 140, appelée simplement platine, peut comporter une bride 141 de commande du déplacement de la platine 140, ou tout autre moyen permettant d'entraîner le déplacement de la platine 140, par traction ou poussée, par rapport au boîtier 130.

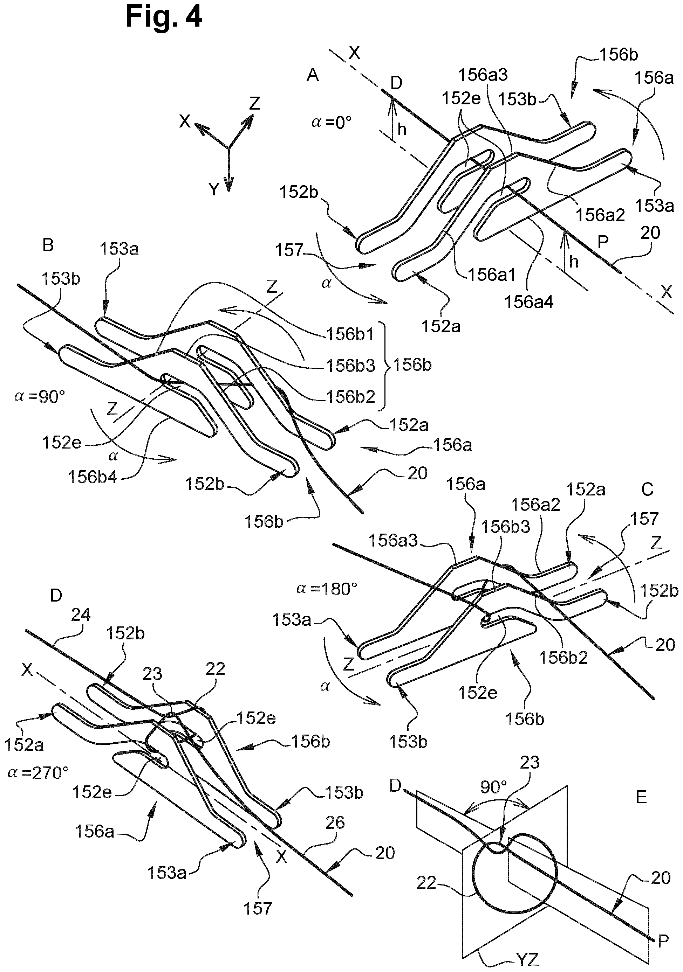

La platine 140 comporte une rainure longitudinale 142, parallèle à l'axe central XX, dans laquelle sont montées une ou plusieurs têtes de bouclage 150 pivotant sous l'action d'une crémaillère. Une tête de bouclage 150, ou tête de mise en forme de boucle, est conçue pour former une boucle 22 avec le fil de ligature 20. Pour cela, chaque tête de bouclage 150 comporte une forme spécifique lui permettant à la fois de maintenir et de guider le fil 20 de sorte que le pivotement de la tête de bouclage entraîne la formation d'une boucle. Cette forme spécifique consiste en une double agrafe 152 et un double croc (153) dans le prolongement l'un de l'autre et montée sur un support pivotant. La figure 4 représente de façon détaillée la double agrafe 152 prolongée par le double croc 153. En particulier, l'ensemble double agrafe et double croc 152-153 comporte deux lames 156a, 156b parallèles l'une à l'autre et espacées l'une de l'autre par une gorge 157 dont la largeur est adaptée pour recevoir le dispositif de nouage 300 décrit par la suite. Les lames 156a, 156b sont symétriques l'une et l'autre par rapport à un plan central longitudinal s'étendant parallèlement auxdites lames, au centre de la gorge 157. La première lame 156a comporte une section sensiblement trapézoïdale avec une face latérale étendue 156a1 qui forme la première agrafe 152a et une autre face latérale étendue 156a2 qui forme le premier croc 153a, chacune des surfaces latérales étendue comprenant une portion en pente, prolongée par une portion plane. La première agrafe 152a et le premier croc 153a sont coplanaires. Symétriquement à la première lame 156a, la deuxième lame 156b comporte une section sensiblement trapézoïdale avec une face latérale étendue 156b1 qui forme la deuxième agrafe 152b et une autre face latérale étendue 156b2 qui forme le deuxième croc 153b, chacune des surfaces latérales étendue comprenant une portion en pente, prolongée par une portion plane. La deuxième agrafe 152b et le deuxième croc 153b sont coplanaires. La double agrafe 152 est formée par la première agrafe 152a et la deuxième agrafe 152b, parallèles l'une à l'autre. De même, le double croc 153 est formé par le premier croc 153a et le deuxième croc 153b, parallèles l'un à l'autre. Chacune des lames 156a, 156b comporte une face supérieure 156a3, 156b3 sensiblement droite et une face inférieure 156a4, 156b4 à partir de laquelle s'étend longitudinalement une fente 152e de retenue du fil 20.

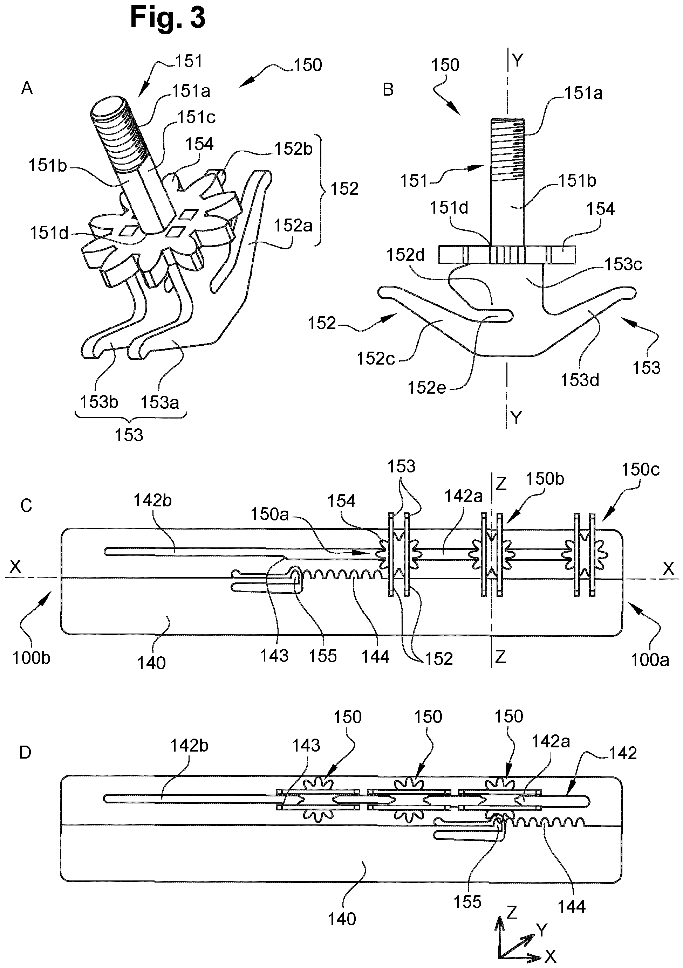

L'ensemble double agrafe et double croc 152-153 est monté sur un support pivotant, par un support permettant la rotation de la tête de bouclage 150. Dans certains modes de réalisation, comme ceux des figures 2 et 3, le support s'étend perpendiculairement aux faces inférieures 156a4, 156b4 de chaque ensemble double agrafe et double croc 152-153. Dans ces modes de réalisation, chaque tête de bouclage 150 peut présenter globalement une forme de marteau avec une jambe et deux pieds s'étendant dans des directions opposées à l'extrémité de la jambe. La jambe de la tête de bouclage 150 est alors formée par une tige 151 et les pieds de la tête de bouclage 150 sont formés par la double agrafe 152 et le double croc 153, montés sur une roue dentée 154.

Dans l'exemple de la figure 2, trois têtes de bouclage 150 sont représentées, étant entendu que l'instrument 100 peut n'en comporter qu'une ou deux ou, au contraire, en comporter plus que trois. En effet, dans une ligature par fil chirurgical, le chirurgien réalise classiquement trois nœuds consécutifs pour assurer un bon maintien de la ligature. Dans le mode de réalisation où l'instrument de ligature 100 comporte trois têtes de bouclage 150, les trois nœuds peuvent être réalisés successivement lors des mêmes opérations et serrés par des opérations distinctes. L'homme du métier comprendra cependant que l'instrument de ligature 100 peut être conçu pour ne former qu'un seul nœud, le chirurgien pouvant alors répéter les opérations de réalisation et de serrage d'un nœud plusieurs fois de suite. Bien entendu, l'instrument de ligature peut comporter également deux ou quatre têtes de bouclage 150 pour réaliser quasi-simultanément, respectivement, deux ou quatre nœuds.

Lorsque l'instrument de ligature comporte plusieurs têtes de bouclage 150, lesdites têtes de bouclage sont montées alignées les unes à la suite des autres dans la rainure longitudinale 142 de la platine. Quel que soit leur nombre, elles sont conçues pour être pivotantes, ou mobiles en rotation, à l'intérieur de la rainure longitudinale 142, leur rotation étant effectuée successivement l'une après l'autre dans un même déplacement de la platine. La rotation des têtes de bouclage 150 est effectuée en commençant par la tête de bouclage distale 150c (c'est-à-dire la plus proche de l'extrémité distale 100b de l'instrument de ligature) et en finissant par la tête de bouclage proximale 150a (c'est-à-dire la plus proche de l'extrémité proximale 100a de l'instrument de ligature). La rotation des têtes de bouclage 150 est obtenue au moyen d'une crémaillère 144 s'engrenant successivement avec la roue dentée 154 de chacune des têtes de bouclage 150. En effet, la crémaillère 144 comporte une pluralité de dents 144a, 144b, 144c, formées le long de la rainure 142 de sorte que le déplacement en translation de la platine 140 entraîne un déplacement de la crémaillère 144 de tête de bouclage en tête de bouclage. Lorsque la crémaillère engrène la roue dentée 154 d'une tête de bouclage 150, son déplacement a pour effet de faire pivoter ladite tête de bouclage.

Dans l'exemple de la figure 2, le boîtier 130 comporte, à proximité de son extrémité distale 100b, un clapet d'alignement 160. Ce clapet d'alignement 160 peut être, par exemple une lame ressort s'étendant entre les deux parois latérales 130a et 130b du boîtier 130 pour fermer ledit boîtier dans sa partie distale proche de l'organe ciblé. Ce clapet d'alignement 160 présente une fonction de porte à fermeture automatique pouvant prendre une position principale fermée et une position partiellement ouverte sous l'effet d'une contrainte dans la direction distale D. En position fermée, comme montré sur le dessin B de la figure 2, le clapet d'alignement 160 a pour fonction de maintenir le fil de ligature 20 afin de lui assurer une certaine tension à l'intérieur du boîtier (et ainsi éviter toute laxité du fil qui pourrait engendrer, par exemple, des boucles non désirées au sein du boîtier). Sous contrainte notamment du dispositif de nouage 300, le clapet d'alignement 160 s'entre-ouvre, comme montré sur le dessin B de la figure 1, et assure le passage du dispositif de nouage 300 pour lui permettre de sortir du boîtier 130 et avancer jusqu'à lacrosse 200 ou, plus précisément, jusqu'à l'extrémité libre du fil 20 et/ou l'organe ciblé. Le clapet d'alignement 160 est initialement dans une position fermée ; il s'entre-ouvre sous l'effet de la poussée du dispositif de nouage 300 lorsque celui-ci se déplace dans la direction distale D. Le clapet d'alignement 160 est donc dimensionné pour assurer une double fonction de maintien du fil 20 et de passage du dispositif de nouage 300.

Une représentation détaillée d'un exemple de tête de bouclage 150 est représentée sur la figure 3. En particulier, les dessins A et B de la figure 3 représentent un exemple d'une tête de bouclage 150, respectivement selon une vue de profil et une vue de côté, inversées, toutes les têtes de bouclage d'un même instrument de ligature étant identiques. Les dessins C et D montrent plusieurs têtes de bouclage 150 montées dans la platine 140. Chaque tête de bouclage 150 est montée par une liaison pivot non-glissante dans le boîtier 130. Dans l'exemple de la figure 3, la liaison pivot non-glissante est obtenue au moyen d'une tige partiellement filetée, étant entendu que d'autres liaisons pivot non-glissantes pourraient être mises en œuvre par l'homme du métier. Dans l'exemple de la figure 3, la liaison pivot non-glissante est réalisée par une tige de fixation 151 ayant une portion filetée 151a et une portion non-filetée 151b, la portion non-filetée étant logée dans la rainure 142 et la portion filetée assurant la fixation de la tige 151 dans le boîtier 130. Pour cela, la portion filetée 151a peut s'étendre à travers la paroi du boîtier 130 et recevoir à son extrémité un écrou de serrage. Alternativement, la tige 151 pourrait être fixée par une goupille ou un circlip ou bien être clipsée sur un épaulement ou une gorge. La portion non-filetée 151b supporte, à son extrémité externe 151d, la roue dentée 154 sur laquelle sont montés l'ensemble double agrafe et double croc 152-153. Cet ensemble double agrafe et double croc 152-153 s'étend perpendiculairement à l'axe YY de la tige 151, dans des directions opposées l'une à l'autre. Autrement dit, dans son état initial, la double agrafe 152 de la tête de bouclage 150 s'étend vers l'une des parois latérales 130a du boîtier 130 tandis que le double croc 153 s'étend vers l'autre parois latérale 130b dudit boîtier.

La double agrafe 152 forme, à l'extrémité de la portion non-fileté 151b de la tige 151, une griffe avec deux doigts 152a, 152b parallèles l'un à l'autre, au cœur de laquelle se loge le fil 20. Autrement dit, la double agrafe 152 présente une section sensiblement en forme de pince avec deux doigts 152c et 152d séparés par un espace en forme de U, appelé fente 152e. Le doigt externe 152c (le plus à l'extrémité de la tête de bouclage) se prolonge au-delà du doigt interne 152d (le plus proche de la portion non-filetée 151b de la tige 151). La fente 152e située entre les deux doigts 152c, 152d forme un logement adapté pour recevoir le fil 20 à l'état initial, avant la formation de la boucle, et adapté pour recevoir et guider le fil 20 pendant la rotation de la tête de bouclage et la mise en forme de boucle dudit fil.

Le double croc 153 forme, à l'extrémité de la portion non-fileté 151b de la tige 151, une fourche à deux dents 153a, 153b parallèles l'une à l'autre et adaptées pour tenir et guider le fil 20. Autrement dit, le double croc 153 présente une section sensiblement en forme de L incliné dont la jambe 153c s'étend sensiblement le long de l'axe YY de la tige 151, ou avec une inclinaison pouvant avoisiner les 45°, et dont le pied 153d s'étend suivant une direction globalement perpendiculaire à l'axe YY, ou avec une inclinaison en direction de la tige 151. Autrement dit, le pied 153d forme, avec la jambe 153c, un angle adapté pour recevoir le fil 20 pendant la formation d'une boucle 22.

La portion non-filetée 151b de la tige 151 est équipée, entre son extrémité 151d et l'ensemble double agrafe et double croc 152-153, d'une roue dentée 154 dont le plan est perpendiculaire à l'axe YY de la tige 151. Autrement dit, la roue dentée 154 s'étend dans un plan XZ parallèle à la surface de la platine 140.

Des têtes de bouclage 150 sont représentées montées sur la platine 140 dans un état initial sur le dessin C de la figure 3 et dans un état final sur le dessin D de la figure 3. En particulier, le dessins C montre trois têtes de bouclage 150, toutes identiques, dans leur état initial, c'est-à-dire avant qu'une boucle ne soit formée. Dans l'exemple du dessin C de la figure 3, le fil 20 n'a pas encore été installé dans l'instrument de ligature 100. Les têtes de bouclage 150 sont alors positionnées parallèlement les unes aux autres, avec leur tige 151 logée dans la rainure longitudinale 142 de la platine 140. Les têtes de bouclage 150 s'étendent alors suivant un axe transversal ZZ, perpendiculaire à l'axe longitudinal XX le long duquel s'étend la rainure 142. Autrement dit, en position initiale, la double agrafe 152 et le double croc 153 s'étendent suivant l'axe ZZ, la double agrafe 152 étant dirigée vers la crémaillère 144, le double croc 153 étant dans le prolongement de la double agrafe 152.

Le fil 20 est installé dans l'instrument de ligature lorsque les têtes de bouclage 150 sont dans leur état initial du dessin C de la figure 3. Il est installé en déroulant le fil 20 depuis une bobine de fil (non visible sur la figure) et le faisant glisser dans les fentes 152e de chacune des têtes de bouclage jusqu'à ce qu'il traverse toutes les têtes de bouclage.

Comme représenté sur le dessin C de la figure 3, la crémaillère 144 est formée dans la platine 140 et comporte des dents 144a, 144b, 144c adaptées pour s'engrener avec les roues dentées 154 de chacune des têtes de bouclage 150. Ainsi, lorsque la platine 140 est déplacée en translation, vers la direction proximale P, la crémaillère 144 entraîne successivement chacune des têtes de bouclage 150 en rotation. La rotation de chaque tête de bouclage correspond à un mouvement rotatif d'environ un trois quarts de tour, c'est-à-dire d'un angle de 270°, positionnant la double agrafe 152 et le double croc 153 alignés le long de l'axe longitudinal XX. Après une rotation d'un 3/4 de tour, la double agrafe 152 de chaque tête de bouclage 150 est positionnée en direction de l'extrémité distale 100b du boîtier 130 tandis que le double croc 153 est positionné en direction de l'extrémité proximale 100a. La double agrafe 152 d'une tête de bouclage 150 fait donc face au double croc 153 de la tête de bouclage 150 voisine. Lorsque l'instrument 100 (ou le boîtier 130) comporte trois têtes de bouclages 150, celles-ci sont alignées les unes à la suite des autres, la double agrafe 152 de la tête de bouclage intermédiaire 150b étant en regard du double croc 153 de la tête de bouclage distale 150a et le double croc 153 de cette même tête de bouclage intermédiaire 150b étant en regard de la double agrafe 152 de la tête de bouclage proximale 150c.

Avant la rotation d'une tête de bouclage 150, le fil 20 est logé dans la fente 152e de la double agrafe 152. Pendant la rotation de cette tête de bouclage 150, le fil 20 est retenu et guidé par l'ensemble double agrafe et double croc 152-153 jusqu'à former une boucle 22 entre les deux agrafes 152a, 152b de la double agrafe 152. La coopération de la double agrafe 152 et du double croc 153 permet la mise en forme de boucle du fil 20. Un exemple de formation d'une boucle de fil est représenté sur la figure 4. Les dessins A, B, C et D de la figure 4 représentent différentes étapes de la formation d'une boucle 22 en fonction de la position de l'ensemble double agrafe et double croc 152-153. Dans la description de la figure 4, les positions du fil 20 autour de la tête de bouclage seront données de la partie distale du fil 20 (ou brin 24) vers la partie proximale de ce même fil 20 (ou brin 26). Le dessin A représente en particulier la position initiale, appelée position A, de l'ensemble double agrafe et double croc 152-153 lorsque les lames 156a, 156b dudit ensemble sont perpendiculaires à l'axe longitudinal XX. Dans cette position A, qui correspond à un angle de 0°, le fil 20 traverse perpendiculairement les fentes 152e et s'étend suivant l'axe longitudinal XX avec une tension définie par le clapet d'alignement 160. Le dessin B représente une première position intermédiaire (dite position B) de l'ensemble double agrafe et double croc 152-153 dans laquelle la tête de bouclage a pivoté d'un angle α de 90° par rapport à la position A (dans le sens anti-horaire). Dans cette position B, le fil 20 s'étend le long du croc 153b de l'ensemble double agrafe et double croc 152-153, traverse les fentes 152e en biais puis s'étend le long de l'agrafe 152a. Le dessin C représente une seconde position intermédiaire (dite position C) de l'ensemble double agrafe et double croc 152-153 dans laquelle la tête de bouclage a pivoté d'un angle de 90° par rapport à la position B, soit un angle α de 180° par rapport à la position A (dans le sens anti-horaire). Dans cette position C, le fil 20 s'étend perpendiculairement aux lames 156a, 156b de l'ensemble double agrafe et double croc 152-153 en appui contre les faces supérieures 156b3, 156a3 dudit ensemble, puis traverse l'ensemble double agrafe et double croc 152-153 via les fentes 152e des agrafes 152b et 152a, et ensuite contourne la double agrafe 152 en appui contre la face latérale 156a2 de l'agrafe 152a et la face latérale 156b2 de l'agrafe 152b jusqu'à être sensiblement perpendiculaire aux lames 156a, 156b de l'ensemble double agrafe et double croc 152-153. Le dessin D représente la position finale (dite position D) de l'ensemble double agrafe et double croc 152-153 dans laquelle la tête de bouclage a pivoté une fois encore d'un angle de 90° par rapport à la position C, soit un angle α de 270° par rapport à la position A (dans le sens anti-horaire). La rotation faisant passer l'ensemble double agrafe et double croc 152-153 de la position C à la position D force le croisement des deux brins de fil 24 (de la portion distale) et 26 (de la portion proximale). Ainsi, dans la position D, le fil 20 forme une boucle 22 qui s'étend en travers de la gorge 157, entre les faces supérieures 156b3, 156a3 de l'ensemble double agrafe et double croc 152-153, les brins 24 et 26 du fil 20 étant colinéaires et s'étendant dans des directions opposées (sensiblement le long de l'axe longitudinal XX) de part et d'autre de la boucle 22. En position D, le fil 20 forme une boucle 22 de forme sensiblement oblongue comportant un entrelacement 23 situé entre les deux faces supérieures 156b3 et 156a3. La boucle 22 globalement oblongue, peut être ovale ou ronde en fonction des dimensions de l'ensemble double agrafe et double croc 152-153, et s'inscrit dans un plan YZ, comme représenté sur le dessin E de la figure 4.

Dans certains modes de réalisation, la rainure longitudinale 142 comporte une largeur (dimension selon l'axe Z) égale sur toute sa longueur (dimension selon l'axe X). Dans d'autres modes de réalisation, comme celui représenté sur les dessins C et D de la figure 3, la rainure longitudinale 142 comporte un étrécissement 143 de sorte qu'une portion large 142a de la rainure 142 comporte une première largeur et qu'une portion étroite 142b de cette même rainure 142 comporte une seconde largeur, inférieure à la première largeur. Cet étrécissement 143 a pour but d'assurer un alignement strict des têtes de bouclage 150 dans leur état final, c'est-à-dire d'assurer une indexation angulaire identique pour toutes les têtes de bouclage. Pour assurer cette indexation angulaire, les tiges 151 de chacune desdites têtes de bouclage peuvent être pourvues d'un méplat 151c dans leur portion non-filetée 151b. Ce méplat 151c assure un positionnement précis et stable de la tige 151 dans la portion étroite 142b de la rainure 142, à partir de l'étrécissement 143. Alternativement, ou en plus de l'étrécissement 143, l'indexation angulaire peut être assurée par un système de dents escamotables sur la crémaillère 144. En effet, la crémaillère 144 de la platine 140 peut être équipée d'une ou plusieurs dents escamotables 155 positionnées à l'extrémité distale de la crémaillère 144. Dans l'exemple de la figure 3, une seule dent escamotable 155 est représentée, cette dent escamotable 155 s'écartant de la roue dentée 154 si une résistance se produit entre ladite roue dentée et la crémaillère. En s'écartant, la dent escamotable 155 libère la roue dentée 154 de la tête de bouclage 150, interrompant ainsi la rotation de ladite tête de bouclage pour lui assurer un positionnement fixe. L'indexation angulaire telle que décrite ci-dessus est réalisée dans le même mouvement que la rotation des têtes de bouclage 150. En effet, les têtes de bouclage 150 étant fixées sur le boîtier 130, le déplacement de la platine 140 entraîne un déplacement de la rainure 142 et de la crémaillère 144, ce qui entraîne la rotation des têtes de bouclage l'une après l'autre. Lorsqu'une tête de bouclage 150 a pivoté d'un angle d'environ 270°, l'étrécissement 143 bloque la tête de bouclage et la/les dents escamotables 155 s'écartent de la roue dentée 154 assurant le blocage définitif de la tête de bouclage. Lorsque toutes les têtes de bouclage 150 ont pivoté et sont bloquées dans leur position finale (parallèle à l'axe longitudinal XX), les têtes de bouclage sont toutes alignées le long du même axe XX de sorte que les boucles 22 formées par chaque tête de bouclage sont également alignées. Toutes les boucles 22 sont identiques et positionnées sur le même axe. Le dispositif de nouage 300 peut alors être utilisé pour former un nœud avec chacune des boucles 22.

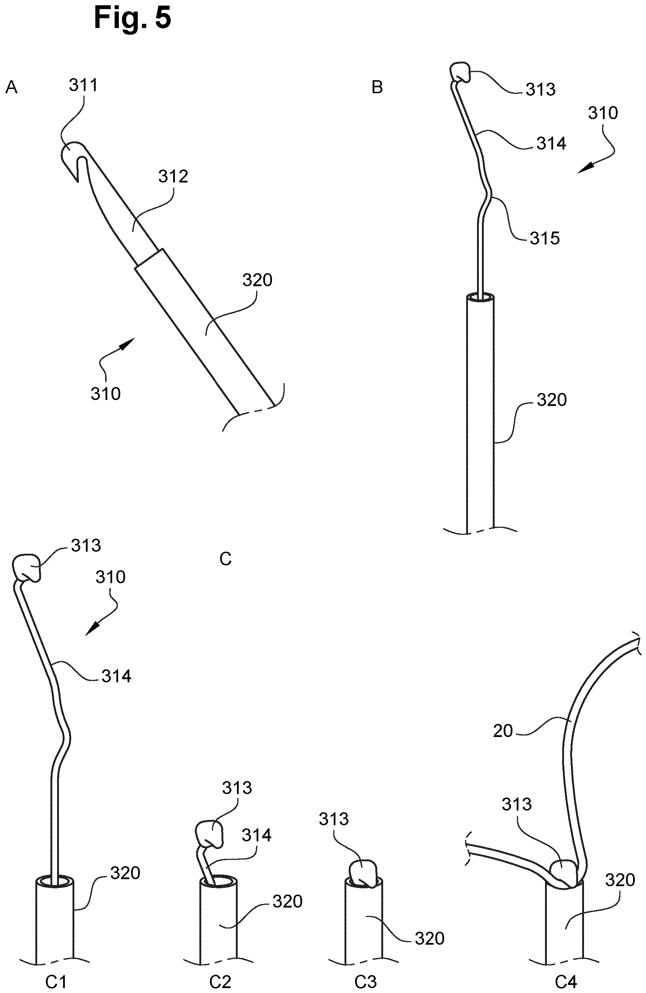

Le dispositif de nouage 300, ou dispositif de formation d'un nœud, est un outil tubulaire adapté pour se déplacer au travers des têtes de bouclage 150, depuis l'extrémité proximale 100a du boîtier 130 jusqu'à l'organe 10 soutenu par la crosse 200. Ce dispositif de nouage 300 a pour fonction d'attraper, ou crocheter, l'extrémité libre du fil 20 et de faire passer cette extrémité du fil 20 dans les boucles de fil 22 formées par les têtes de bouclage 150 afin de former des nœuds. Pour cela, le dispositif de nouage 300 comporte un crochet 310 monté coulissant à l'intérieur d'un premier fourreau 320. On appelle « crochet » un dispositif d'attache en partie recourbé pour permettre la préhension du fil 20. Des exemples de crochet 310 sont représentés sur la figure 5. Le crochet 310 comporte une tige 312 à l'extrémité de laquelle est fixé un moyen de crochetage 311, 313 du fil 20, c'est-à-dire un moyen permettant d'accrocher le fil 20 pour le déplacer à l'intérieur du boîtier 130 afin de finaliser la formation les nœuds de ligature.

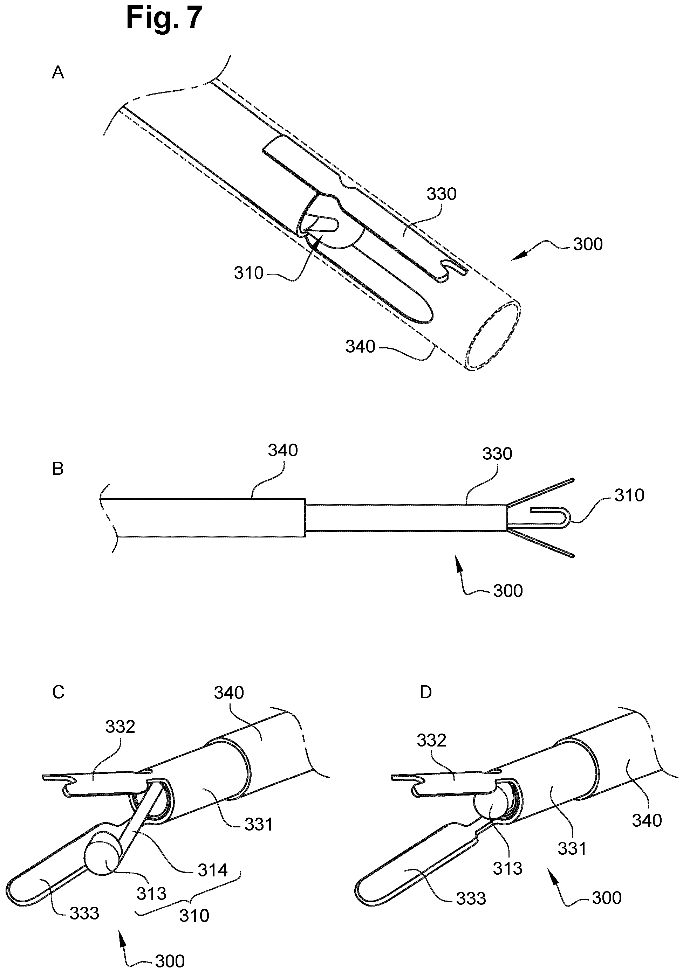

Un premier exemple de crochet 310 est représenté sur le dessin A de la figure 5. Dans cet exemple, le crochet 310 comporte une tringle sensiblement rectiligne 312 à l'extrémité distale de laquelle est montée une attache recourbée 311. L'attache recourbée 311 peut présenter une forme en U (comme dans l'exemple du dessin B de la figure 7) ou une forme de bec (comme dans l'exemple du dessin A de la figure 5) permettant d'accrocher le fil 20 à l'extérieur du boîtier 130 pour ensuite l'amener à l'intérieur dudit boîtier et notamment dans les boucles de fil 22 préalablement réalisées par les têtes de bouclage 150.

Un deuxième exemple de crochet 310 est représenté sur le dessin B de la figure 5. Dans cet exemple, le crochet 310 comporte une tringle coudée 314 munie, à son extrémité distale, d'un renflement 313. La tringle coudée 314 peut, par exemple, être pourvue d'une angulosité 315 formant un coude dans une zone de la tringle relativement proche du renflement 313 (l'angulosité est plus proche de l'extrémité distale de la tringle que de son extrémité proximale).

Quel que soit le mode de réalisation du crochet 310, l'extrémité proximale de la tringle du crochet (non visible sur la figure 5) est fixée au moyen de commande 120 par lequel le déplacement du crochet 310 est commandé.

Dans l'exemple du dessin C de la figure 5, le crochet 310, dans son mode de réalisation avec une tringle coudée, est représenté dans plusieurs positions de l'opération de crochetage, ou préhension, du fil 20. Le dessin C1 montre le crochet 310 partiellement sorti de son fourreau 320 (appelé premier fourreau) avec son renflement 313 sensiblement désaxé par rapport à l'axe traversant le fourreau 320 ; le dessins C2 montre le crochet 310 lorsque sa tringle 314 est quasiment rentrée dans le fourreau 320, le renflement 313 étant alors sensiblement aligné sur l'axe traversant le fourreau 320 ; le dessin C3 montre le crochet 310 lorsque le renflement 313 est positionné à l'entrée du fourreau 320 ; le dessin C4 montre une position du crochet similaire à celle du dessin C3 mais lorsque le crochet 310 a crocheté l'extrémité du fil 20 et que l'extrémité du fil 20 est dans l'entrée du fourreau 320, retenu entre le renflement 313 et ledit fourreau. L'extrémité du fil 20 ainsi retenue par le crochet 310 et le premier fourreau 320 peut être déplacée à travers le boîtier 130. L'extrémité du fil 20 est, en particulier, translatée vers les têtes de bouclage 150 de façon à passer au travers de chacune des boucles de fil 22 formées autour des doubles agrafes 152 desdites têtes de bouclage. Une fois l'extrémité du fil 20 passée à travers une boucle de fil 22, un nœud 25 est réalisé. En passant l'extrémité du fil 20 dans toutes les boucles de fil 22 formées par les têtes de bouclage 150, tous les nœuds 25 sont formés lors d'une même opération, c'est-à-dire d'un même déplacement de l'outil de nouage 300, ces nœuds étant alors non-serrés et positionnés autour des doubles agrafes 152 des têtes de bouclage.

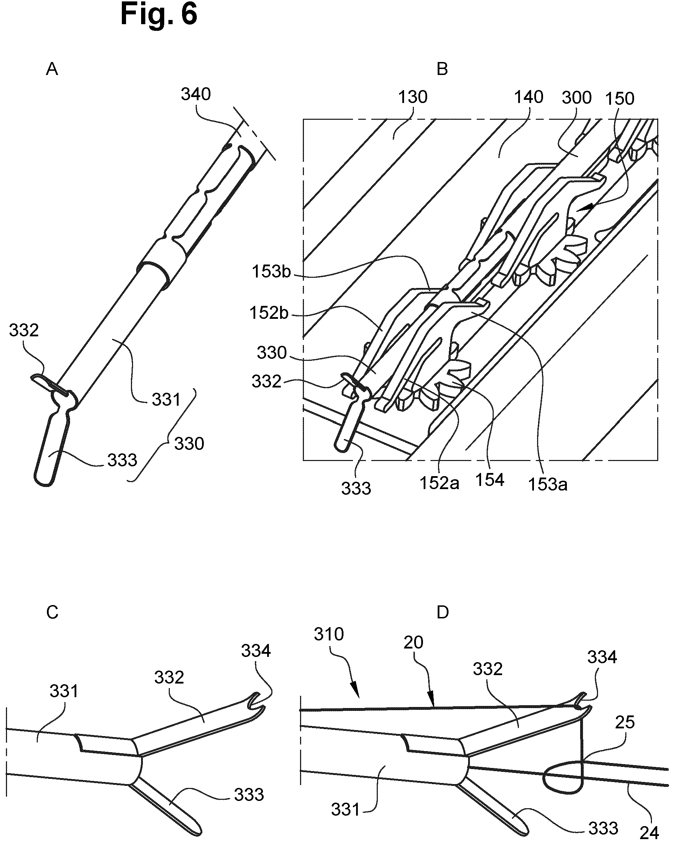

Outre sa fonction de crochetage du fil 20, le dispositif de nouage 300 a également des fonctions de décrochage et de serrage des nœuds. Pour cela, le dispositif de nouage 300 comporte une broche-poussoir 330 représentée sur la figure 6 et conçue pour décrocher les nœuds des têtes de bouclage 150 et serrer ces nœuds contre l'organe ciblé. La broche-poussoir 330 est un outil tubulaire comportant deux ailettes 332, 333 montées à l'extrémité distale d'un bras 331 coulissant à l'intérieur d'un fourreau 340, appelé deuxième fourreau. Les deux ailettes 332, 333 sont positionnées de part et d'autre de l'extrémité distale du bras 331. Elles sont montées escamotables à l'extrémité du bras 331 pour pouvoir se loger à l'intérieur du fourreau 340. Plusieurs exemples d'une broche-poussoir 330 sont représentés sur les dessins A, C et D de la figure 6. En position escamotée, les deux ailettes 332, 333 sont en regard l'une de l'autre et s'étendent dans le prolongement du bras 331, à l'intérieur du deuxième fourreau 340. En position ouverte, comme montré sur le dessin A, les deux ailettes 332 et 333 sont écartées l'une de l'autre, de part et d'autre de l'axe central du bras 331, formant ainsi une fourche pouvant décrocher et pousser un nœud 25. Lors du décrochement d'un nœud 25, les deux ailettes 332, 333 se positionnent de part et d'autre d'un nœud, l'extrémité du bras 331 située entre les deux ailettes se plaquant contre l'entrelacement 23 du fil 20, entre les deux agrafes 152a, 152b de la double agrafe 152 de sorte à pousser le nœud 25 hors de la double agrafe 152. Dans certains modes de réalisation, comme ceux des dessins C et D de la figure 6, au moins une des ailettes présente à son extrémité libre une encoche 334 permettant de serrer le nœud vers l'organe ciblé. En effet, les ailettes 332, 333 ont pour rôle de décrocher le nœud 25 et de le pousser le long du brin central 24 du fil qui s'étend dans les gorges 157 des têtes de bouclage 150. Une des ailettes, voire les deux, comporte une encoche 334 dont la fonction est principalement d'appliquer un effort sensiblement perpendiculaire à l'axe du brin central 24, cet effort assurant le serrage du nœud 25. Chacune des ailettes 332, 333 peut être formée par une lamelle plate ou concave et au moins une des ailettes comporte une extrémité sensiblement en forme de V formant l'encoche 334.Comme montré sur le dessin D de la figure 6, l'encoche 334 permet d'attraper le fil du nœud 25 pour le tirer dans une direction perpendiculaire à celle du brin central 24 du fil. Du fait de leur forme, les ailettes 332, 332 sont relativement flexibles. Les ailettes 332, 332 sont, en outre, réalisées dans un matériau flexible, comme l'inox, le Nitinol ou le polyamide, leur permettant, d'une part, de s'escamoter à l'intérieur du deuxième fourreau 340 et, d'autre part, de s'écarter l'une de l'autre et se plaquer contre l'organe ciblé pour permettre le serrage du nœud 25 contre l'organe.

Comme représenté sur le dessin B de la figure 6, la broche-poussoir 330 est un outil longiligne adapté pour se déplacer au travers des têtes de bouclage 150, entre les agrafes 152a, 152b de la double agrafe 152 et les deux crocs 153a, 153b du double croc 153. En effet, comme montré sur le dessin B, l'espace entre les agrafes 152a, 152b et les crocs 153a, 153b forme une gorge 157 à travers les têtes de bouclage 150. Cette gorge 157, ou chemin, est rectiligne et bordé, d'un côté, par l'agrafe 152a et le croc 153a et, de l'autre côté, par l'agrafe 152b et le croc 153b de ces mêmes têtes de bouclage 150. Le chemin ainsi formé au sein des têtes de bouclage 150 permet au dispositif de nouage 300 de se déplacer longitudinalement à travers le boîtier 130. Ce chemin à travers les têtes de bouclage 150 permet non seulement le déplacement en translation de la broche-poussoir 330 et du crochet 310 mais également l'accès aux boucles de fil 22 formées à l'intérieur desdites têtes de bouclage. Il permet ainsi au dispositif de nouage 300, dans un premier mouvement, de crocheter l'extrémité du fil 20 puis, dans un deuxième mouvement, de faire passer l'extrémité du fil 20 dans les boucles de fil 22 pour former les nœuds 25 (au moyen du crochet 310). Il permet ensuite, dans une succession de troisièmes mouvements, de décrocher un par un chacun des nœuds des doubles agrafes 152 (au moyen de la broche-poussoir 330) et de serrer chaque nœud contre l'organe ciblé.

Dans un mode de réalisation préféré, le crochet 310 et la broche-poussoir 330 constituent un seul et même outil assurant à la fois les fonctions du crochet et celles de la broche-poussoir. Pour cela, le bras 331 de la broche-poussoir est creuse et le crochet 310 avec son fourreau 320 sont montés dans le bras 331 qui, à son tour, est monté dans le fourreau 340. Autrement dit, le crochet 310 est logé dans le premier fourreau 320, qui lui-même est logé dans le bras creux 331, qui à son tour est logé dans le deuxième fourreau 340. Le crochet 310, le premier fourreau 320, la broche-poussoir 330 et le deuxième fourreau 340 sont donc tous concentriques et ne forment qu'un unique outil de nouage. Le crochet 310 et la broche-poussoir 330 sont reliés chacun au dispositif de commande 120 de façon à pouvoir être commandés indépendamment l'un de l'autre. Le chirurgien peut donc utiliser alternativement le crochet 310, la broche-poussoir 330 ou les deux à la fois. Pour assurer l'indépendance du crochet et de la broche-poussoir, un élément de retenue, tel qu'un clip, peut être prévu au sein du fourreau 340 pour retenir la broche-poussoir ou le crochet en fonction des besoins.

Dans une variante améliorée, représentée sur la figure 7, le premier fourreau 320 est supprimé, le bras creux 331 assurant la fonction de ce premier fourreau, à savoir la fonction de fourreau du crochet 310. Dans cette variante, le crochet 310 coulisse à l'intérieur du bras creux 331 comme dans un fourreau. Le crochet 310 est donc logé dans le bras creux 331 à l'extrémité duquel s'étendent les ailettes 332 et 333, la broche-poussoir 330 et ses ailettes 332, 333 coulissant à l'intérieur du deuxième fourreau 340. Le dessin A de la figure 7 représente cette variante améliorée du dispositif de nouage 300, dans une position fermée où le crochet 310, le bras creux 331 et les ailettes 332, 333 sont tous positionnés à l'intérieur du deuxième fourreau 340. Dans cette position fermée, le dispositif de nouage 300 peut coulisser à l'intérieur du boîtier 130 sans qu'aucune de ses fonctions ne soit utilisée, par exemple pour son déplacement allant de l'extrémité proximale 100a du boîtier 130 jusqu'à la crosse 200 pour l'opération de crochetage dudit fil. Les dessins B et C représentent deux exemples de ce dispositif de nouage 300 lorsque les ailettes 332, 333 sont déployées et que le crochet 310 est en train de sortir du bras creux 331. Le dessin D représente ce dispositif de nouage 300 lorsque seules les ailettes 332, 333 sont déployées, le crochet 310 étant totalement logé dans le bras creux 331.

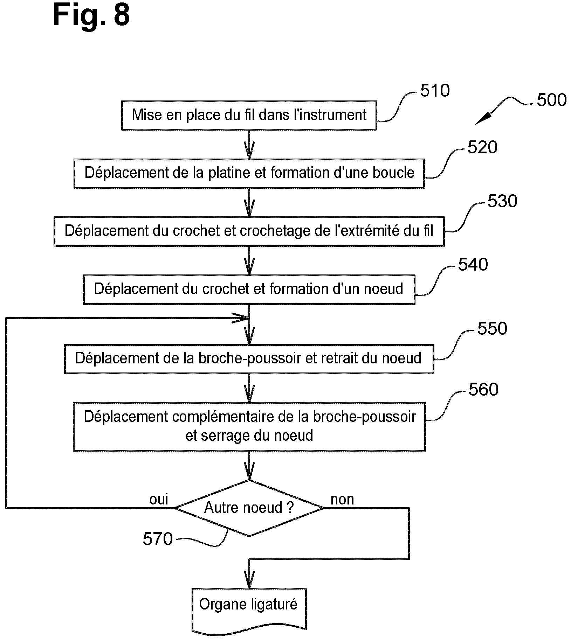

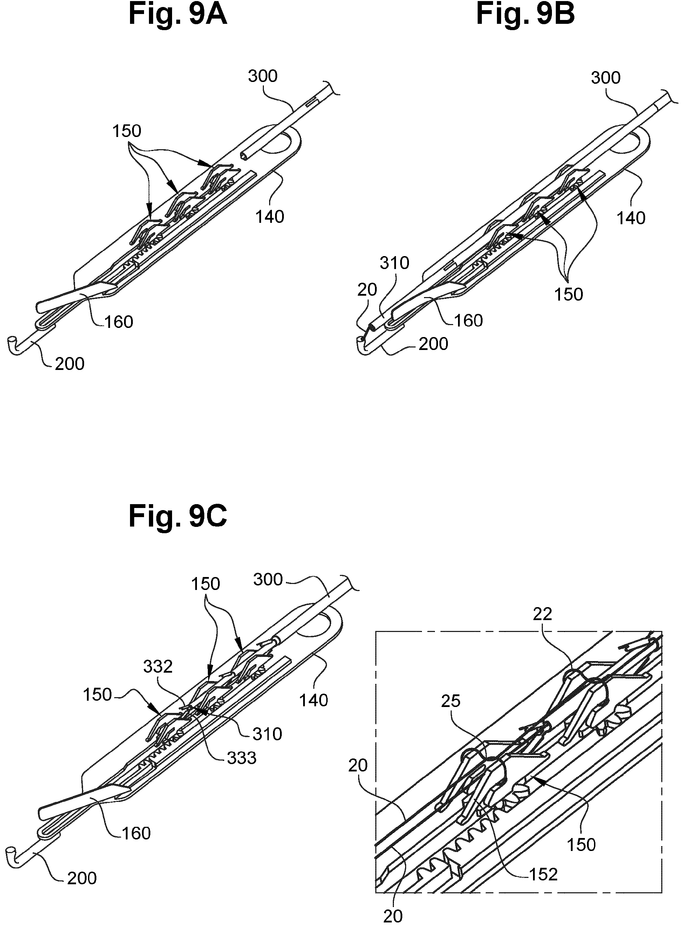

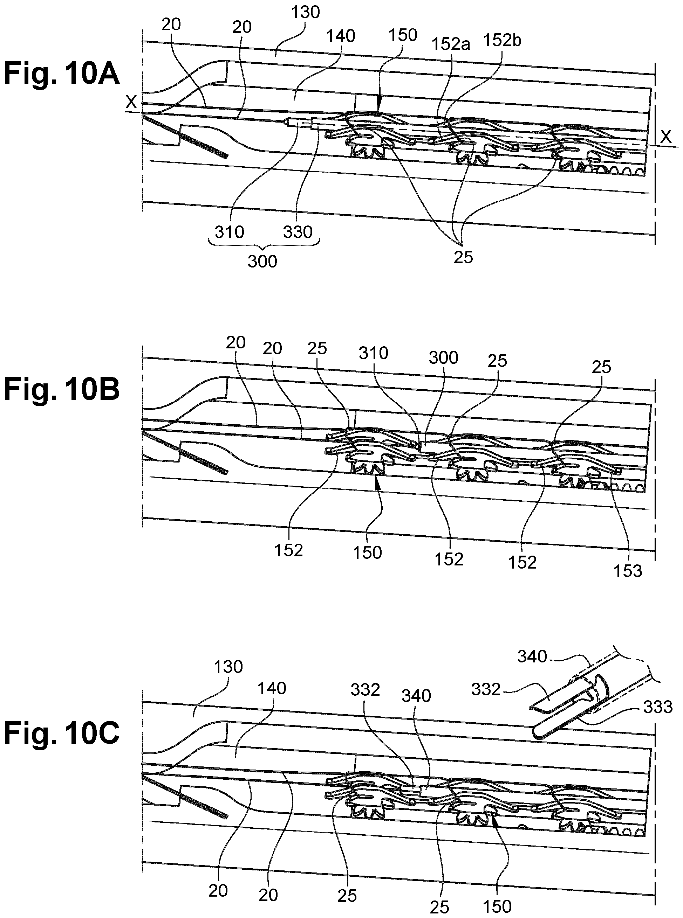

Le procédé de mise en œuvre de l'instrument de ligature 100 décrit ci-dessus est représenté sur la figure 8 par des blocs fonctionnels. L'instrument de ligature 100 est représenté schématiquement sur les figures 9A à 9E durant certaines étapes du procédé 500, l'instrument 100 étant alors représenté de façon simplifiée (sans étui, ni boîtier) pour une meilleure compréhension du procédé. Pour certaines étapes du procédé 500, l'instrument de ligature est également représenté sur les figures 10A à 10E avec une représentation schématique du fil 20, pour une meilleure compréhension du procédé.

Le procédé 500 comporte une première opération 510 de mise en place initiale du fil de ligature 20 à l'intérieur de l'instrument de ligature 100, avant la formation de nœuds de ligature 25. Initialement, une bobine d'un fil chirurgical standard est installée à proximité de l'extrémité proximale 100a du boîtier 130 et maintenue par un dispositif d'attache temporaire tel que des clips (non visibles sur les figures). Le fil 20 est tiré de la bobine de fil et engagé latéralement dans la ou les têtes de bouclage 150 à l'intérieur du boîtier 130. En particulier, le fil 20 est engagé dans la fente 152e des doubles agrafes 152 de chacune des têtes de bouclage 150, les doubles agrafes 152 étant dans leur position initiale, perpendiculaire à l'axe central XX. L'engagement latéral du fil 20 dans les doubles agrafes peut être effectué manuellement par le chirurgien ou un opérateur. Alternativement, il peut être effectué au moyen d'un dispositif à bascule ayant sensiblement une forme de peigne permettant de pousser le fil 20 horizontalement vers les doubles agrafes 152. Lorsque l'instrument de ligature 100 comporte un étui 110, comme dans l'exemple de la figure 1, l'étui 110 est ouvert, au moins dans sa partie entourant les têtes de bouclage 150, lors de l'insertion du fil 20 dans le boîtier 130. La fermeture de l'étui 110 peut entraîner un mouvement latéral de translation du fil 20 pour l'engager dans les doubles agrafes 152. Une fois le fil 20 engagé dans les têtes de bouclage 150, l'extrémité libre du fil 20 est positionnée dans la rainure centrale de la crosse 200 qui assure un maintien suffisant pour permettre l'enroulement du fil 20 autour de l'organe ciblé tout en permettant un décrochement aisé de l'extrémité du fil 20 au moyen du crochet 310. Lorsque le fil 20 est mis en place, autour de l'organe ciblé et dans les têtes de bouclage 150, le clapet d'alignement 160 positionné près de l'extrémité distale 100b du boîtier 130 est fermé, ce qui permet une tension du fil 20 dans la zone proximale et la zone centrale où se trouvent les têtes de bouclage 150, sans que cette tension ne soit transmise dans la zone distale notamment la zone proche de la crosse 200. Le clapet d'alignement 160 bloque ainsi la translation du fil 20 de l'extrémité distale 100b vers l'extrémité proximale 100a mais autorise sa translation de l'extrémité proximale vers l'extrémité distale, notamment pour permettre la mise en place du fil dans les têtes de bouclage.

Le procédé 500 comporte ensuite une opération 520 de déplacement en translation de la platine 140, le long de l'axe central XX, en direction de l'extrémité proximale 100a de l'instrument de ligature 100. Cette translation entraîne un déplacement de la crémaillère 144 qui entraîne à son tour, successivement, la rotation de la roue dentée 154 de chacune des têtes de bouclage 150 et, par conséquence, la rotation de la tête de bouclage 150 elle-même. Toutes les têtes de bouclages 150 sont ainsi entraînées en rotation successivement, les unes après les autres, dans un même déplacement de la platine 140. La rotation de chaque tête de bouclage 150 (rotation d'un ¾ de tour) a pour effet de créer une boucle 22 dans le fil 20. Une fois la boucle de fil 22 réalisée, celle-ci est maintenue sur la tête de bouclage 150 par les agrafes 152a, 152b de la double agrafe 152.

Le procédé 500 comporte ensuite une opération 530 de déplacement du dispositif de nouage 300 à l'intérieur du boîtier 130. Plus précisément, le crochet 310 du dispositif de nouage 300 est déplacé longitudinalement (c'est-à-dire parallèlement à l'axe central XX) à l'intérieur du boîtier 130 en direction de l'extrémité distale 100b de l'instrument de ligature 100 jusqu'à sortir dudit boîtier 130 et atteindre l'extrémité libre du fil 20 maintenue par la crosse 200. Le crochet 310 accroche alors l'extrémité libre du fil 20 et la ramène jusqu'au premier fourreau 320 où elle est maintenue entre le moyen de crochetage 311 ou 313 et ledit fourreau 320. L'état initial du crochet 310, avant tout déplacement, est représenté sur la figure 9A. Le crochet 310, au moment du crochetage de l'extrémité libre du fil 20, est représenté sur la figure 9B.

Une fois l'extrémité libre du fil 20 crochetée et maintenue entre le crochet 310 et le premier fourreau 320, le procédé 500 se poursuit par une opération 540 de déplacement longitudinal du dispositif de nouage 300 dans les gorges 157 des têtes de bouclage 150, en direction de l'extrémité proximale 100a dudit boîtier 130. Le recul du dispositif de nouage 300 (c'est-à-dire son déplacement vers l'extrémité proximale) maintenant l'extrémité libre du fil 20 permet de faire passer ladite extrémité du fil 20 à travers chacune des boucles de fil 22 formées dans les têtes de bouclage 150. Le passage de l'extrémité du fil 20 dans une boucle de fil 22 assure la finalisation de la formation d'un nœud 25 correspondant. Le recul du crochet 310 avec la formation d'un nœud 25 est représenté sur la figure 9C ainsi que sur les figures 10A et 10B. Les figures 10A et 10B représentent, en particulier, le crochet 310 dans le mode de réalisation où il est monté à l'intérieur de la broche-poussoir 330 : les ailettes 332, 333 de la broche-poussoir sont alors escamotées à l'intérieur du fourreau et le moyen de crochetage 311 ou 313 tient l'extrémité du fil 20 pour la faire passer à travers les boucles de fil 22.

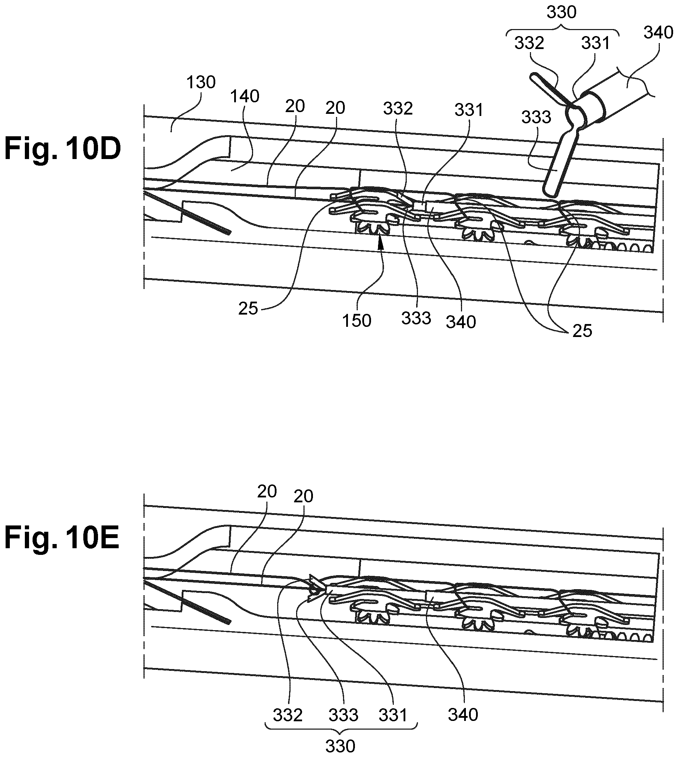

Le procédé 500 comporte ensuite une nouvelle opération de déplacement longitudinal du dispositif de nouage 300 à l'intérieur du boîtier 130. Cette opération 550 de déplacement consiste à déplacer le dispositif de nouage 300 en direction de l'extrémité distale 100b de l'instrument de ligature 100 pour décrocher les nœuds 25 formés dans les têtes de bouclage. Dans cette opération 550, la broche-poussoir 330 est déplacée, de l'extrémité proximale 100a vers l'extrémité distale 100b du boîtier 130, pour décrocher et pousser successivement chacun des nœuds ; le crochet 310 avec son fourreau 320 reste alors en position proximale pour maintenir le brin central 24 le long duquel le nœud 25 est poussé. La broche-poussoir 330 est de préférence coulissée, dans sa position fermée, c'est-à-dire avec les ailettes 332, 333 escamotées dans le fourreau 340, comme représenté sur la figure 10C. Une fois la broche-poussoir 330 à proximité d'un nœud 25, les ailettes 332, 333 sont extraites du fourreau 340 et déplacées dans la direction distale pour pousser le nœud hors de la double agrafe 152 et, ainsi, le décrocher de la tête de bouclage 150. Un exemple d'une broche-poussoir 330 avec les ailettes 332, 333 escamotées est représenté sur la figure 10C. Un exemple d'une broche-poussoir 330 avec les ailettes 332, 333 écartées est représenté sur la figure 10D.

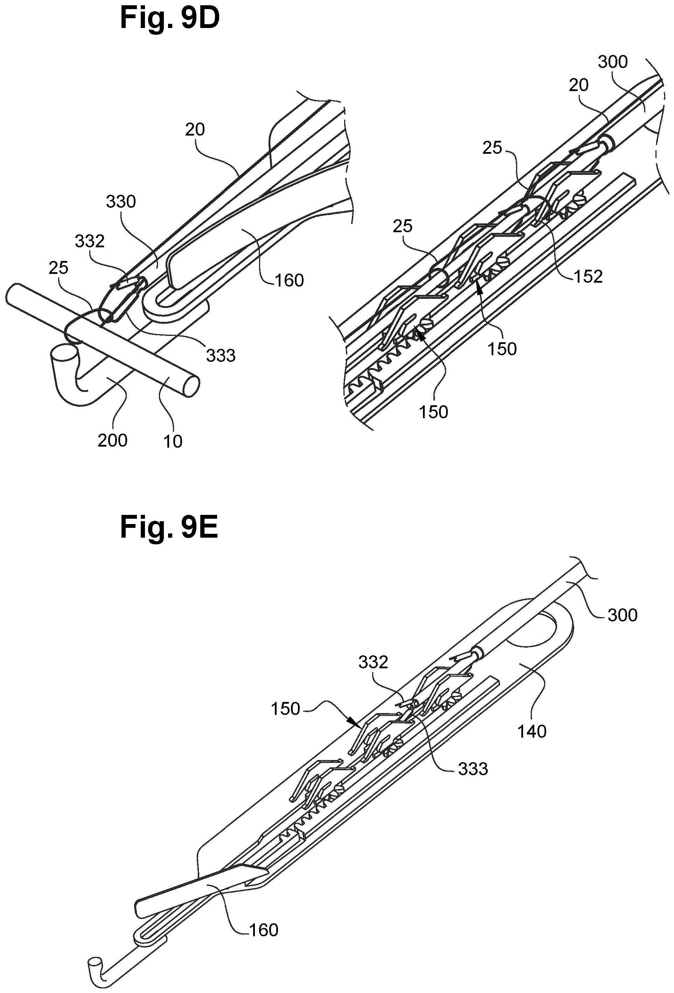

Le procédé 500 comporte ensuite une opération 560 de déplacement complémentaire du dispositif de nouage 300 pour serrer chacun des nœuds contre l'organe ciblé. Cette opération 560 consiste à déplacer la broche-poussoir 330 longitudinalement dans la direction distale D jusqu'à sortir du boîtier 130 afin de positionner le nœud 25 au contact de l'organe ciblé et le serrer contre ledit organe au moyen notamment de l'ailette à encoche 334. L'opération de poussage et de serrage du nœud est réalisée au moyen des ailettes 332, 333. Le serrage d'un premier nœud 25 est représenté sur la figure 9D et la figure 10E. Une fois le nœud poussé et serré, la broche-poussoir 330 est reculée vers la direction proximale P.

Dans un mode de réalisation, l'instrument de ligature 100 comporte autant de têtes de bouclage 150 que de nœuds à former et serrer contre l'organe ciblé. Dans un mode de réalisation préféré, l'instrument de ligature 100 comporte trois têtes de bouclage 150a, 150b, 150c. L'instrument de ligature 100 permet alors de former successivement les trois nœuds dans un même déplacement du dispositif de nouage 300.. Ainsi, trois boucles de fil 22 sont formées pendant une même opération 520 et les trois nœuds 25 correspondant à ces boucles sont formés au cours de la même opération 540.

Les trois nœuds 25 sont ensuite décrochés, poussés et serrés l'un après l'autre au cours de trois déplacements 550 et 560 distincts. Autrement dit, les opérations 550 et 560 sont réitérées pour chaque nœud 25 de la façon suivante :
- la broche-poussoir 330 coulisse jusqu'à la tête de bouclage distale 150c où elle décroche, puis pousse et serre le nœud 25 de la tête de bouclage 150c contre l'organe ciblé ;
- la broche-poussoir 330 revient jusqu'à la tête de bouclage intermédiaire 150b où elle décroche, puis pousse et serre le nœud 25 de la tête de bouclage 150b contre le nœud précédent ; un exemple de recul de la broche-poussoir 330 est représenté sur la figure 9E ; et
- la broche-poussoir 330 revient jusqu'à la tête de bouclage proximale 150a où elle décroche, puis pousse et serre le nœud 25 de la tête de bouclage 150a contre les deux nœuds précédents.

Une fois les trois nœuds serrés, l'organe est ligaturé.

Dans un autre mode de réalisation, comme celui représenté sur la figure 8, l'instrument de ligature 100 comporte une unique tête de bouclage 150. Les nœuds 25 sont alors formés, décrochés et serrés séquentiellement, nœud par nœud. Autrement dit, un premier nœud 25 est formé en effectuant les opérations 520, 530, 540, 550 et 560. Lorsque le premier nœud est formé, le procédé 500 comporte une étape 570 consistant à vérifier si un autre nœud est prévu. Si c'est le cas et qu'un deuxième nœud est prévu, alors les opérations 520, 530, 540, 550 et 560 sont réitérées jusqu'à ce que le deuxième nœud soit serré contre le premier nœud. Un troisième nœud (voire un quatrième nœud ou autant de nœuds que décidé par le chirurgien) peut ensuite être réalisé de la même façon que le deuxième nœud. L'organe ciblé est considéré comme ligaturé quand tous les nœuds prévus ont été réalisés.

Ainsi, l'instrument de ligature 100 selon l'invention permet au chirurgien de réaliser autant de nœuds que nécessaire pour ligaturer l'organe ciblé, tous les nœuds étant identiques avec un serrage régulier. Le procédé de mise en œuvre décrit montre que la réalisation de ces nœuds est simple et ne nécessite que des déplacements en translation de la platine et du dispositif de nouage.

Bien que décrits à travers un certain nombre d'exemples, variantes et modes de réalisation, l'instrument de ligature et son procédé de mise en œuvre comprennent divers variantes, modifications et perfectionnements qui apparaîtront de façon évidente à l'homme du métier, étant entendu que ces variantes, modifications et perfectionnements font partie de la portée de l'invention.

## Revendications

1. Instrument de ligature (100) d'un organe (10) d'un corps humain ou animal au moyen d'un fil de ligature (20), comportant :
- un bâti (130) sur lequel est montée au moins une tête de bouclage (150) supportant le fil de ligature (20),
- un actionneur (140) assurant une rotation de la tête de bouclage par rapport au bâti, la rotation de la tête de bouclage mettant le fil de ligature en forme de boucle (22) autour de ladite tête de bouclage,
- une crosse (200) fixée à l'extérieur du bâti et maintenant une extrémité libre du fil de ligature (20) à proximité de l'organe à ligaturer, et
- un dispositif de nouage (300), tubulaire, se déplaçant au travers de la tête de bouclage et assurant la préhension de l'extrémité libre du fil de ligature et la formation d'un nœud (25) par passage de ladite extrémité libre du fil de ligature au travers de la boucle préalablement formée.

2. Instrument de ligature selon la revendication 1, **caractérisé en ce qu'**il comporte au moins deux têtes de bouclage (150a, 150b, 150c) alignées, assurant la formation d'au moins deux boucles de fil de ligature, alignées, formées par un unique actionnement de l'actionneur, le dispositif de nouage assurant la formation d'au moins deux nœuds par passage de l'extrémité libre du fil de ligature au travers de chacune des deux boucles.

3. Instrument de ligature selon la revendication 1 ou 2, **caractérisé en ce que** la tête de bouclage (150), montée pivotante dans le bâti, comporte une double agrafe (152) et un double croc (153) dans le prolongement l'un de l'autre, la rotation de la double agrafe et du double croc entraînant une rotation et un guidage du fil de ligature lui assurant une mise en forme de boucle (22).

4. Instrument de ligature selon la revendication 3, **caractérisé en ce que** la tête de bouclage comporte deux lames (126a, 126b), parallèles et symétriques l'une et l'autre par rapport à un plan central longitudinal, chaque lame comportant une section sensiblement trapézoïdale dont une première face latérale étendue (156b1, 156a1) forme une agrafe (152a, 152b) et une deuxième face latérale étendue (156b2, 156a2) forme un croc (153a, 153b), l'agrafe et le croc étant coplanaires.

5. Instrument de ligature selon la revendication 4, **caractérisé en ce que** chaque lame (126a, 126b) comporte une face supérieure (126a3, 126b3) sensiblement droite et une face inférieure (126a4, 126b4) à partir de laquelle s'étend longitudinalement une fente (152e) de retenue du fil de ligature.

6. Instrument de ligature selon l'une quelconque des revendications 4 à 5, **caractérisé en ce que** les deux lames (126a, 126b) de la double agrafe (152) et du double croc (153) forment une gorge (157) s'étendant suivant un axe longitudinal (XX), dans laquelle se déplace le dispositif de nouage (300).

7. Instrument de ligature selon l'une quelconque des revendications 1 à 6, **caractérisé en ce que** l'actionneur (140) comporte un dispositif de pivotement (144) de la tête de bouclage entraînant la rotation d'un ¾ de tour de la tête de bouclage depuis une position initiale (A) perpendiculaire à un axe longitudinal (XX) vers une position finale (D) parallèle à l'axe longitudinal

8. Instrument de ligature selon l'une quelconque des revendications précédentes, **caractérisé en ce que** le dispositif de nouage (300) comporte un crochet (310) monté coulissant à l'intérieur d'un premier fourreau (320) et assurant la préhension de l'extrémité libre du fil de ligature (20) et la formation du nœud (25) par passage de l'extrémité dudit fil à travers la boucle (22) formée par la tête de bouclage.

9. Instrument de ligature selon la revendication 8, **caractérisé en ce que** le crochet (310) comporte une attache recourbée (311), montée à une extrémité distale d'une tringle sensiblement rectiligne (312).

10. Instrument de ligature selon la revendication 8, **caractérisé en ce que** le crochet (310) comporte une tringle coudée (314), équipée d'un renflement (313) à son extrémité distale.

11. Instrument de ligature selon l'une quelconque des revendications précédentes, **caractérisé en ce que** le dispositif de nouage (300) comporte une broche poussoir (330) dont une extrémité distale est équipée de deux ailettes (332, 333), escamotables à l'intérieur d'un deuxième fourreau (340), les deux ailettes formant une fourche de décrochage du nœud.

12. Instrument de ligature selon les revendications 8 et 11, **caractérisé en ce que** le crochet (310), le premier fourreau (320), la broche poussoir (330) et le deuxième fourreau (340) sont montés concentriques les uns dans les autres de sorte à former un unique outil déplaçable au travers de la tête de bouclage.

13. Instrument de ligature selon les revendications 8 et 11, **caractérisé en ce que** la broche poussoir (330) comporte une tige (331) creuse formant le premier fourreau dans lequel coulisse le crochet (310).

14. Instrument de ligature selon l'une quelconque des revendications précédentes, **caractérisé en ce que** le bâti (130) comporte, à une extrémité distale, un clapet d'alignement (160) du fil de ligature autorisant le déplacement du fil de ligature dans la direction distale et le blocage dudit fil de ligature dans la direction proximale.

15. Procédé (500) de mise en œuvre d'un instrument de ligature (100) selon l'une quelconque des revendications précédentes, comportant les opérations suivantes :
a) mise en place (510) d'un fil de ligature (20) à l'intérieur de l'instrument de ligature, le fil traversant la tête de bouclage (150) avec une tension prédéterminée, une extrémité libre du fil étant installée sur une crosse (200) à une extrémité distale (100b) dudit instrument de ligature et une bobine de fil étant positionnée au voisinage d'une extrémité proximale (100a) dudit instrument de ligature,
b) commande de l'actionneur pour entraîner la tête de bouclage (150) en rotation de sorte à former une boucle du fil de ligature (22),
c) déplacement du dispositif de nouage (530) au travers de la boucle formée par la tête de bouclage en direction de l'extrémité distale (100b) de l'instrument de ligature et préhension de l'extrémité libre du fil (20),
d) déplacement, en direction de l'extrémité proximale (100a) de l'instrument de ligature, du dispositif de nouage (540) et de l'extrémité libre du fil de ligature au travers de la boucle de fil de ligature pour former un nœud (25),
e) déplacement du dispositif de nouage (550) au travers de la tête de bouclage, en direction de l'extrémité distale (100b) de l'instrument de ligature, et décrochage du nœud (25) hors de la tête de bouclage (150),
f) déplacement complémentaire (560) du dispositif de nouage (300) en direction de l'organe à ligaturer et serrage du nœud (25) contre ledit organe à ligaturer (10).
